# EUROPEAN PATENT SPECIFICATION

(11) **EP 2 873 433 B1**
(45) Date of publication and mention of the grant of the patent: **18.05.2016**
(21) Application number: 13192727.9
(22) Date of filing: 13.11.2013
(51) Int. Cl.: A61M 16/04, A61M 16/08, F16K 11/076, F16K 11/085, F16K 35/02

(54) **SWITCHABLE ADAPTER FOR A SUCTION TUBE**
SCHALTBARER ADAPTER FÜR EINEN SAUGSCHLAUCH
ADAPTATEUR COMMUTABLE DESTINÉ À UN TUBE D'ASPIRATION

(43) Date of publication of application: 20.05.2015
(73) Proprietor: Vitaltec Corporation, Taichung City (TW)
(72) Inventor: Chiu, Sheng-Yu, Houli Dist. Taichung City (TW)
(74) Representative: Pallini Gervasi, Diego

(56) References cited:
- WO-A2-2004/011829
- US-A- 5 181 508
- US-A- 5 427 145
- US-A1- 2004 221 852
- US-A1- 2010 147 310
- US-A1- 2011 155 135

## Description

### 1. Field of the Invention

The present invention relates to an adapter for a suction tube, especially to a switchable adapter for a suction tube.

### 2. Description of the Prior Art(s)

According to medical practice guidelines, when a patient is unable to swallow, to discharge sputa, or to breathe autonomously, or when respiratory disorders occur, an incision has to be made on an anterior portion of the neck of the patient in order to put a tracheotomy tube into a trachea of the patient through the incision. The tracheotomy tube is selectively connected to an oxygen supply apparatus, an open suction tube, or a closed suction tube via an adapter in order to assist the patient with breathing or to suck the sputa out of the trachea of the patient.

A conventional adapter for a suction tube, such as an ISOLATION VALVE FOR A CLOSED SUCTION DEVICE disclosed in U.S. patent publication No. 2011/0155135, has a body and a valve. The body has a communicating channel, a patient end connector, a breathing circuit connector and a suction tube connector. The communicating channel has a chamber and an opening. The patient end connector, the breathing circuit connector and the suction tube connector are radially formed on and protrude from the external surface of the communicating channel and communicate with the chamber of the communicating channel. The valve is rotatably connected to the body and has an isolation pipe and a control button. The isolation pipe is rotatably mounted in the chamber of the communicating channel via the opening and has a communicating hole, an inlet hole and a sucking hole. The control button is formed on the isolation pipe above the top of the communicating channel.

Another conventional adapter for a suction tube, such as a RESPIRATORY CARE ASSEMBLY disclosed in U.S. patent No. 6,612,304, comprises a manifold and an adapter. The manifold has an internal channel, multiple access ports, and a supporting component. The access ports of the manifold communicate with the internal channel and are respectively connected to the trachea of the patient and the oxygen supply apparatus. The supporting component has an opening communicating with the internal channel. The adapter is rotatably mounted on the supporting component and has two access ports respectively connected to the closed suction tube and the open suction tube. When the adapter is rotated, the adapter is switched, such that the two access ports of the adapter alternatively align with the opening of the supporting component to communicate with the internal channel of the manifold. While one of the access ports of the adapter communicates with the internal channel, the other one of the access ports of the adapter is separated from the internal channel.

However, when the adapter is rotated, exterior air may flow into the trachea of the patient through interstices between the adapter and the supporting component, putting the patient at the risk of infection. Moreover, since there are no alignment structures formed on the adapter and the supporting component, the medical personnel cannot be ensured whether the access port of the adapter has been aligned with the opening of the supporting component or not. Furthermore, the adapter has to be rotated 180 degrees to allow the other access port of the adapter to align with the opening of the supporting component, which is time-consuming and causes discomfort of the patient.

The main objective of the present invention is to provide a switchable tube for a suction tube.

Therefore, according to the present invention, a switchable adapter for a suction tube according to claim 1 is provided.

The switchable adapter has a manifold, an air-tight component, a switching base, and a cover.

The manifold has a main tube, at least one branch tube, and a pivot portion. The main tube has an internal channel defined and extending between a front end of the main tube and a rear end of the main tube. The at least one branch tube is formed on and protrudes from the main tube, and communicates with the internal channel of the main tube. The pivot portion is attached to the rear end of the main tube and has a side recess and a mounting hole. The side recess is formed in an outer surface of the pivot portion. The mounting hole is formed through an inner sidewall defined in the side recess and communicates with the internal channel of the main tube.

The air-tight component is made of resilient material and has a main portion tightly fitted in the side recess of the pivot portion. The main portion of the air-tight component has a through hole aligning with and communicating with the internal channel of the main tube.

The switching base is pivotally connected to the pivot portion of the manifold and has a bottom panel, an arced sidewall, and two connecting tubes. The arced sidewall is formed on and protrudes up from the bottom panel. The pivot portion of the manifold is mounted in a pivot chamber surrounded by the arced sidewall of the switching base. The connecting tubes perpendicularly protrude from the arced sidewall and alternatively align with the through hole of the air-tight component to communicate with the internal channel of the main tube.

The cover is mounted on an end of the pivot portion of the manifold and is disposed opposite to the bottom panel of the switching panel.

An included angle defined between the two connecting tubes is only 30 to 90 degrees, such that rotating the switching base 30 is convenient and time-saving. Accordingly, chance of causing discomfort to the patient is greatly reduced. Furthermore, with the air-tight component tightly fitted in the side recess of the pivot portion, no exterior air would flow into the internal channel of the manifold through interstices between the pivot portion and the switching base, thereby preventing the patient from being infected.

### IN THE DRAWINGS:

Fig. 1 is an operational perspective view of a switchable adapter for a suction tube in accordance with the present invention, shown connecting with a medicine feeding device and a closed suction tube;
Fig. 2 is an exploded perspective view of the switchable adapter in Fig. 1;
Fig. 3 is an exploded perspective view of a manifold and an air-tight component of the switchable adapter in Fig. 1;
Fig. 4 is a cross-sectional side view of the switchable adapter in Fig. 1;
Fig. 5 is an operational cross-sectional side view of the switchable adapter in Fig. 1;
Fig. 6 is a cross-sectional top view of the switchable adapter in Fig. 1;
Fig. 7 is an operational cross-sectional top view of the switchable adapter in Fig. 1;
Fig. 8 is another operational cross-sectional top view of the switchable adapter in Fig. 1;
Fig. 9 is an operational perspective view of the switchable adapter in Fig. 1, showing a main cap caps a second connecting tube;
Fig. 10 is an operational top view of the switchable adapter in Fig. 1, showing the second connecting tube is used for receiving an open suction tube; and
Fig. 11 is an operational top view of the switchable adapter in Fig. 1, showing the main cap is removed from the second connecting tube.

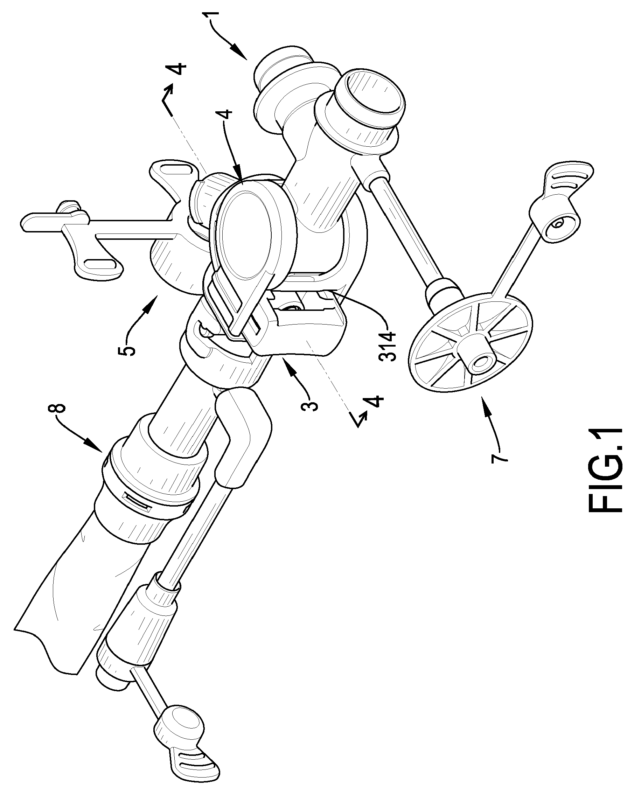

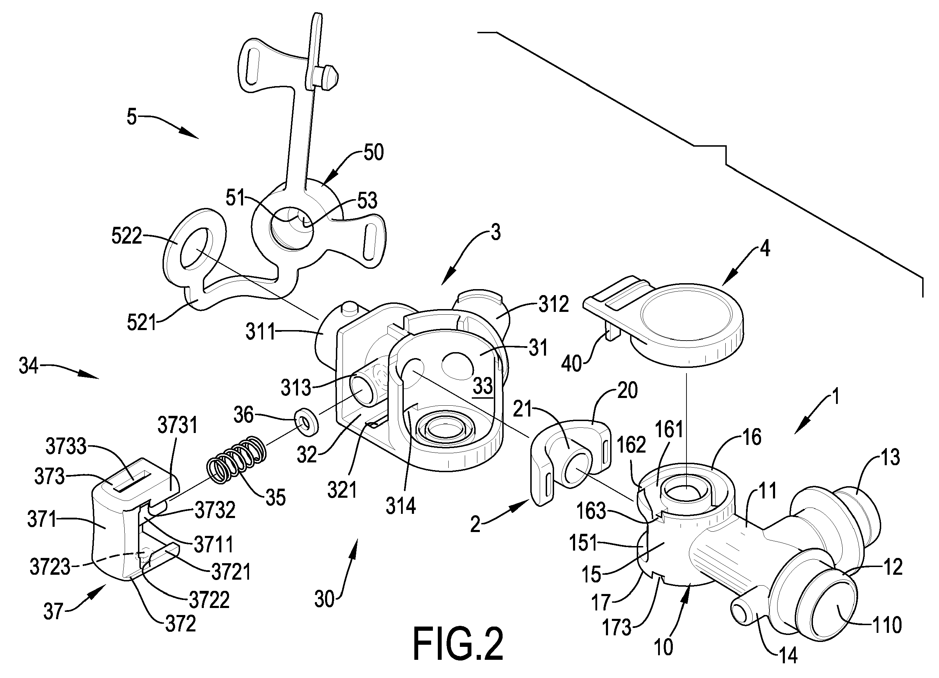

With reference to Figs. 1 and 2, a switchable adapter for a suction tube in accordance with the present invention comprises a manifold 1, an air-tight component 2, a switching base 30, a pressing assembly 34, a cover 4, and a cap set 5.

The manifold 1 has a main tube 11, at least one branch tube 12, 13, 14, and a pivot portion 10. The main tube 11 has a front end, a rear end, and an internal channel 110. The internal channel 110 is defined and extends between the front and the rear ends of the main tube 11. The at least one branch tube 12, 13, 14 is formed on and protrudes from the main tube 11, and communicates with the internal channel 110 of the main tube 11. Specifically, the manifold 1 has multiple branch tubes 12, 13, 14 including a trachea tube 12, an oxygen tube 13, and a medicine tube 14. The trachea tube 12 protrudes coaxially with the main tube 11 from the front end of the main tube 11. The oxygen tube 13 and the medicine tube 14 protrude perpendicularly from the main tube 11.

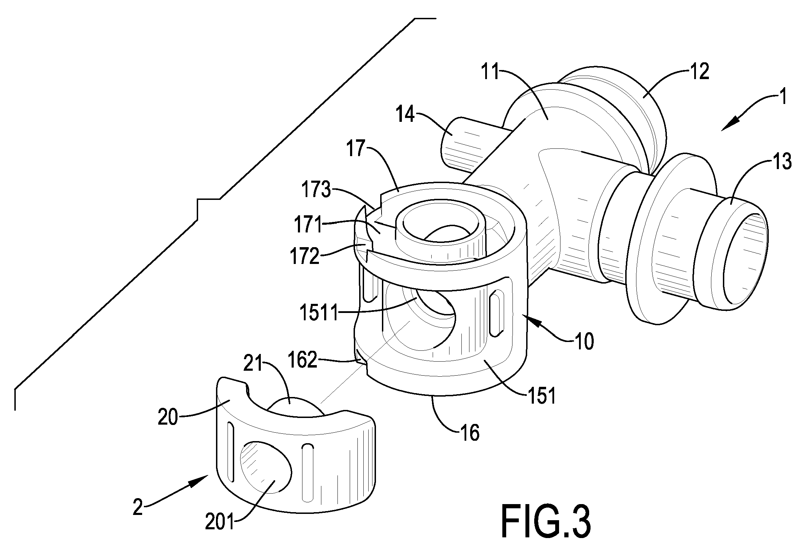

With further reference to Fig. 3, the pivot portion 10 is attached to the rear end of the main tube 11, and has an outer surface 15, an upper end 16, a lower end 17, a side recess 151, an inner sidewall, a mounting hole 1511, an upper recess 161, an upper peripheral sidewall, a lower recess 171, and a lower peripheral sidewall. The outer surface 15 of the pivot portion 10 is attached to the rear end of the main tube 11. The side recess 151 is formed in the outer surface 15 of the pivot portion 10. The inner sidewall of the pivot portion 10 is defined in the side recess 151. The mounting hole 1511 is formed through the inner sidewall of the pivot portion 10 and communicates with the internal channel 110 of the main tube 11.

The upper recess 161 is formed in the upper end 16 of the pivot portion 10. The upper peripheral sidewall is defined around the upper recess 161 and has a first notch 162 and a second notch 163. The lower recess 171 is formed in the lower end 17 of the pivot portion 10. The lower peripheral sidewall is defined around the lower recess 171 and has a first notch 172 and a second notch 173. The first notch 172 of the lower peripheral sidewall corresponds in position to the first notch 162 of the upper peripheral sidewall. The second notch 173 of the lower peripheral sidewall corresponds in position to the second notch 163 of the upper peripheral sidewall.

The air-tight component 2 is made of resilient material, such as resilient plastic, and has a main portion 20 and a side tube 21. The main portion 20 may be arced, is tightly fitted in the side recess 151 of the pivot portion 10, and has a through hole 201 aligning with and communicating with the internal channel 110 of the main tube 11. Specifically, the main portion 20 is slightly larger than the side recess 151 of the pivot portion 10 in size, such that the main portion 20 is tightly fitted in the side recess 151 of the pivot portion 10. The side tube 21 protrudes from the main portion 20, is disposed around the through hole 201 of the main portion 20, protrudes in the mounting hole 1511 of the pivot portion 10, and communicates with the internal channel 110 of the main tube 11.

The switching base 30 is connected to the pivot portion 10 of the manifold 1 and has a bottom panel 32, an arced sidewall 31, a pivot chamber 33, at least one connecting tube 311, 312, and a mounting tube 313. The bottom panel 32 has a guiding slot 321 being elongated.

The arced sidewall 31 is formed on and protrudes up from the bottom panel 32, and has an inner arc surface, an outer arc surface, and a mounting slot 314. The mounting slot 314 is formed through the arced sidewall 31, is disposed beside the bottom panel 32, and selectively corresponds in position to the first or the second notch 172, 173 of the lower peripheral sidewall of the pivot portion 10. The pivot chamber 33 is defined above the bottom panel 32, is surrounded by the arced sidewall 31, and receives the air-tight component 2 and the pivot portion 10 with the lower end 17 of the pivot portion 10 facing the bottom panel 32.

The at least one connecting tube 311, 312 perpendicularly protrudes from the outer arc surface of the arced sidewall 31 and communicates with the pivot chamber 33. Specifically, the switching base 30 has two connecting tubes 311, 312. The two connecting tube 311, 312 alternatively align with the through hole 201 of the air-tight component 2 to communicate with the internal channel 110 of the main tube 11. The two connecting tubes 311, 312 include a first connecting tube 311 and a second connecting tube 312. The first connecting tube 311 extends along a first axis. The second connecting tube 312 extends along a second axis. An included angle A defined between the first axis and the second axis is 30 to 90 degrees.

The mounting tube 313 perpendicularly protrudes from and communicates with the first connecting tube 311, corresponds in position to and extends parallel to the guiding slot 321 of the bottom panel 32, and has a distal end.

The pressing assembly 34 is mounted on the mounting tube 313 of the switching base 30, and has a pressing element 37 and a resilient element 35.

The pressing element 37 is disposed beside the distal end of the mounting tube 313 of the switching base 30 and has a force exertion portion 371, a guiding shaft 3711, and two guiding sidewalls 372, 373. The guiding shaft 3711 protrudes from the force exertion portion 371, is mounted in the mounting tube 313 of the switching base 30, and selectively protrudes into the first connecting tube 311. The guiding sidewalls 372, 373 separately protrude from the force exertion portion 371, and are oppositely disposed beside and parallel to the guiding shaft 3711. Each of the guiding sidewalls 372, 373 has an inner surface, an elongated edge, and a guiding wing 3721, 3731. The inner surface of the guiding sidewall 372, 373 faces the guiding shaft 3711. The elongated edge of the guiding sidewall 372, 373 corresponds in position to the arced sidewall 31 of the switching base 30. The guiding wing 3721, 3731 protrudes from the elongated edge of the guiding sidewall 372, 373, and has a rear end. The rear end of the guiding wing 3721, 3731 is disposed apart from the force exertion portion 371 to form a gap 3722, 3732 defined between the rear end of the guiding wing 3721, 3731 and the force exertion portion 371.

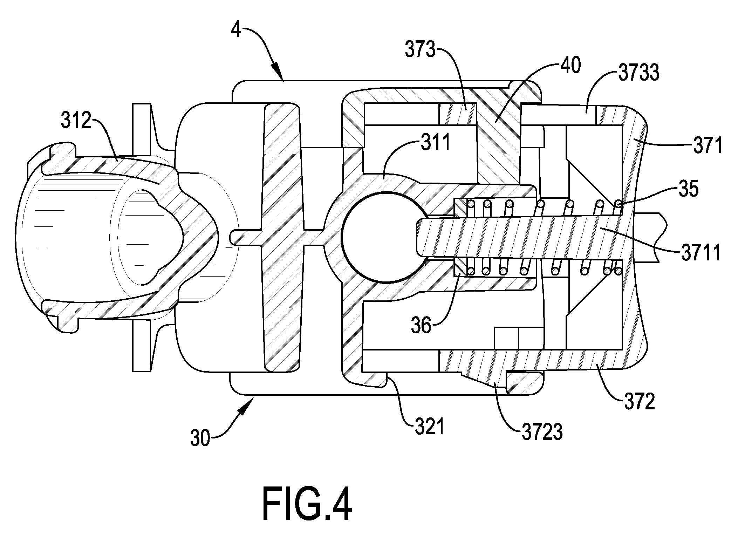

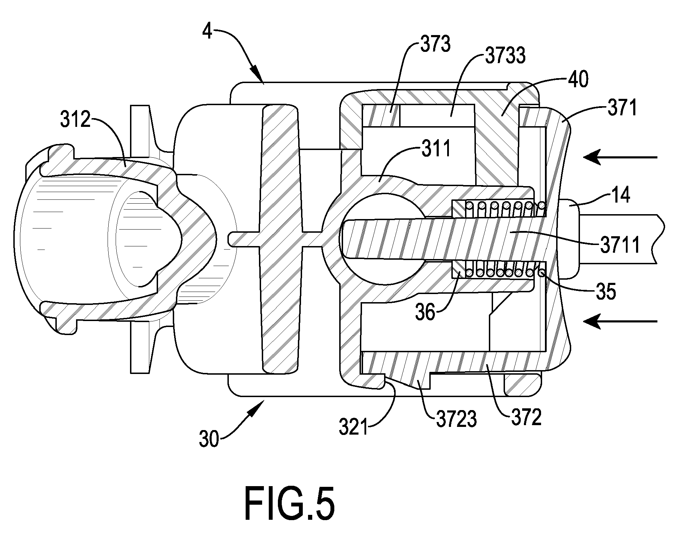

With further reference to Figs. 4 and 5, specifically, the two guiding sidewalls 372, 373 include a first guiding sidewall 372 and a second guiding sidewall 373. The first guiding sidewall 372 further has a sliding protrusion 3723 formed on the inner surface of the first guiding sidewall 372 and protruding in the guiding slot 321 of the bottom panel 32. The guiding wing 3721 of the first guiding sidewall 372 is mounted through the mounting slot 314 of the arced sidewall 31, protrudes into the lower recess 171 of the pivot portion 10, and selectively engages in the first or the second notch 172, 173 of the lower peripheral sidewall of the pivot portion 10.

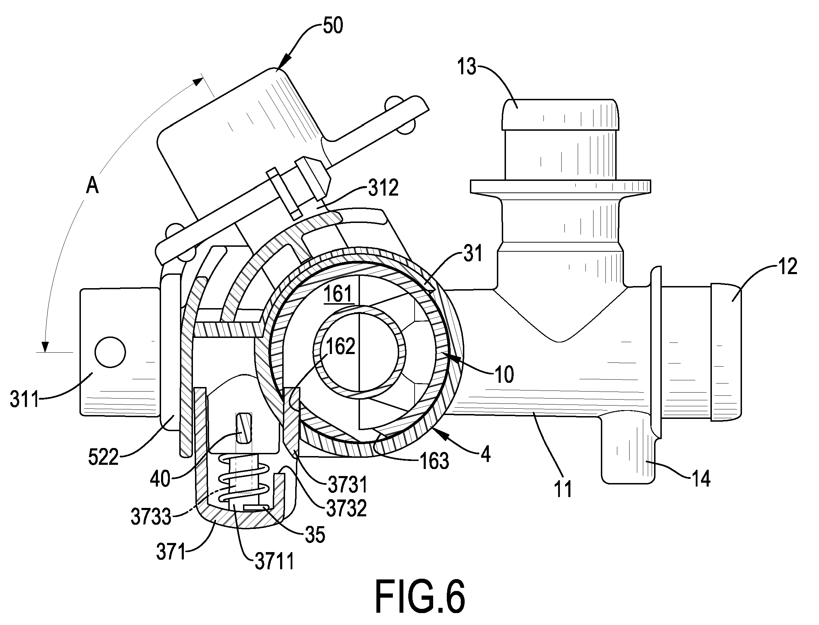

With further reference to Fig. 6, the second guiding sidewall 373 further has a sliding slot 3733. The sliding slot 3733 is elongated and is formed through the second guiding sidewall 373. The guiding wing 3731 of the second guiding sidewall 373 protrudes into the upper recess 161 of the pivot portion 10 and selectively engages in the first or the second notch 162, 163 of the upper peripheral sidewall of the pivot portion 10.

The resilient element 35 is mounted in the mounting tube 313 of the switching base 30, is mounted around the guiding shaft 3711 of the pressing element 37, and has two opposite ends respectively abutting the first connecting tube 311 and the force exertion portion 371 of the pressing element 37 to push the pressing element 37 to slide away from the switching base 30. Specifically, the resilient element 35 is a compression spring. With the resilient element 35 pushing the pressing element 37, the guiding wings 3721, 3731 securely engage in the first or the second notches 162, 163, 172, 173 of the pivot portion 10 of the manifold 1, such that the switching base 30 is held at a specific angular position relative to the pivot portion 10.

Preferably, the pressing assembly 34 further has a pad 36 mounted in the mounting tube 313 of the switching base 30. The two opposite ends of the resilient element 35 respectively abut the pad 36 and the force exertion portion 371 of the pressing element 37. The guiding shaft 3711 of the pressing element 37 is selectively mounted through the pad 36 to protrude into the first connecting tube 311.

The cover 4 is mounted on the upper end 16 of the pivot portion 10 of the manifold 1 and has an inner surface and a limiting protrusion 40. The limiting protrusion 40 protrudes from the inner surface of the cover 4 and protrudes in the sliding slot 3733 of the pressing element 37. With the limiting protrusion 40 of the cover 4 protruding in the sliding slot 3733 of the pressing element 37 and with the sliding protrusion 3723 of the pressing element 37 protruding in the guiding slot 321 of the bottom panel 32, a sliding range of the pressing element 37 is limited.

The cap set 5 is connected to the first connecting tube 311 of the switching base 30 and selectively caps a distal end of the second connecting tube 312 of the switching base 30. The cap set 5 includes a main cap 50, a sealing film 53, and a collar 522. The main cap 50 caps the distal end of the second connecting tube 312 and has a through hole 51 aligning with the second connecting tube 312. The sealing film 53 is mounted in the through hole 51 of the main cap 50 and has a peripheral edge partially attached to the main cap 50. Thus, the sealing film 53 selectively seals the through hole 51 of the main cap 50. The collar 522 is mounted around the first connecting tube 311 of the switching base 30 and is connected with the main cap 50 via a connecting strip 521.

As shown in Fig. 1, the trachea tube 12 is used to connect with a trachea of a patient, the oxygen tube 13 is used to connect with an oxygen supply system, and the medicine tube 14 is used to connect with a medicine feeding device 7.

With further reference to Fig. 6, when the first connecting tube 311 communicates with the internal channel 110 of the main tube 11, the guiding wings 3721, 3731 of the first and the second guiding sidewalls 372, 373 of the pressing element 37 engage in the first notches 172, 162 of the lower and the upper peripheral sidewalls of the pivot portion 10, and the second connecting tube 312 is separated from the internal channel 110 of the main tube 11. The first connecting tube 311 is used to connect with a closed suction tube 8. Thus, since the first connecting tube 311 communicates with the internal channel 110 of the main tube 11, the suction tube 8 can suck sputa out of the trachea of the patient.

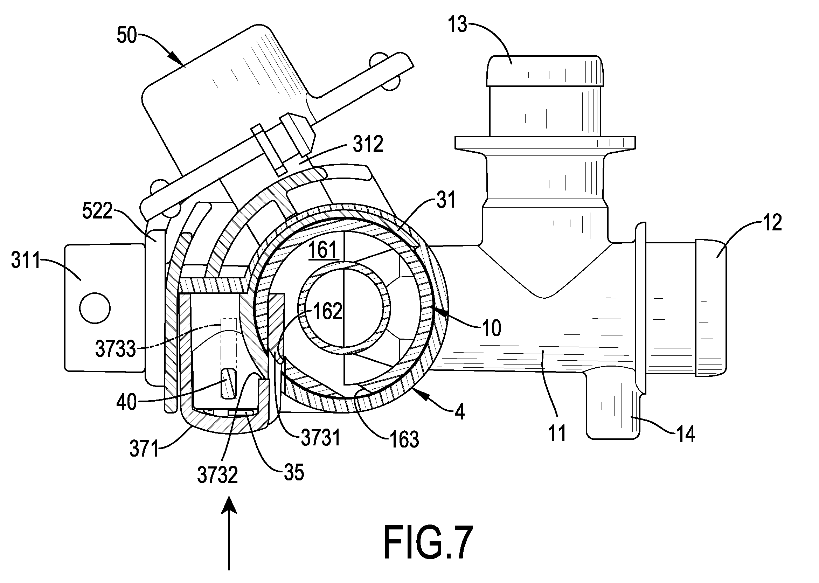

With further reference to Fig. 7, before switching the switching base 30 to allow the second connecting tube 312 to communicate with the internal channel 110 of the main tube 11, the pressing element 37 is pressed toward the first connecting tube 311, the resilient element 35 is compressed, and the guiding wings 3721, 3731 of the first and the second guiding sidewalls 372, 373 of the pressing element 37 disengage from the first notches 172, 162 of the lower and the upper peripheral sidewalls of the pivot portion 10. Thus, the switching base 30 is rotatable relative to the pivot portion 10 of the manifold 1.

When the switching base 30 is rotated, the lower peripheral sidewall and the upper peripheral side wall of the pivot portion 10 protrude in and engage respectively the gaps 3722, 3723 of the pressing element 37, such that the resilient element 35 cannot push the pressing element 37 backwardly.

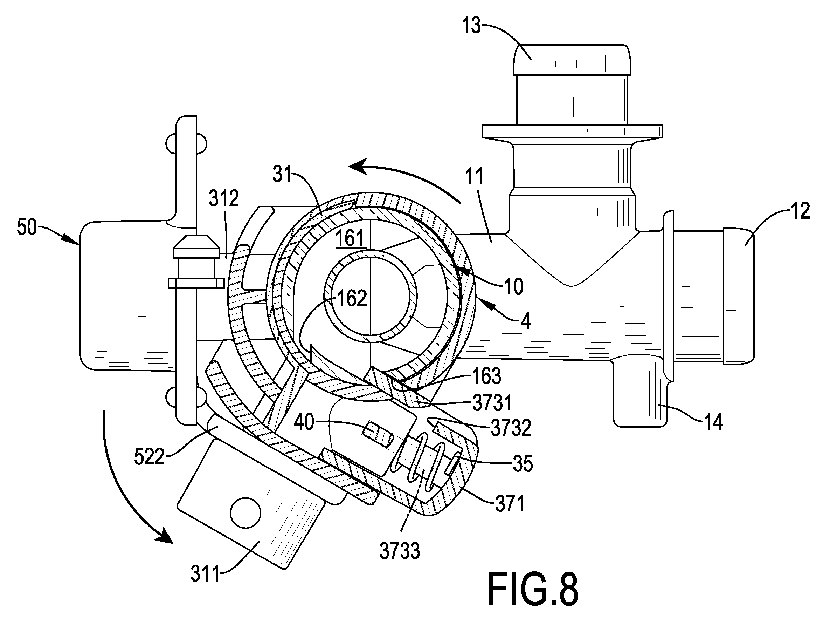

With further reference to Fig. 8, as the switching base 30 is rotated to allow the second connecting tube 312 to align with the through hole 201 of the air-tight component 2 and to communicate with the internal channel 110 of the manifold 1, the lower peripheral sidewall and the upper peripheral side wall of the pivot portion 10 respectively disengage from the gaps 3722, 3723 of the pressing element 37.

Thus, the resilient element 35 pushes the pressing element 37 backwardly, and the guiding wings 3721, 3731 of the first and the second guiding sidewalls 372, 373 of the pressing element 37 engage in the second notches 173, 163 of the lower and the upper peripheral sidewalls of the pivot portion 10.

With the guiding wings 3721, 3731 of the pressing element 37 selectively engage in the first or the second notches 172, 162, 173, 163 of the pivot portion 10, an operator can be sure that the first connecting tube 311 or the second connecting tube 312 has been communicating with the internal channel 110 of the manifold 1. Moreover, since the included angle A defined between the first connecting tube 311 and the second connecting tube 312 is only 30 to 90 degrees, rotating the switching base 30 is convenient and time-saving. Accordingly, the chance of causing discomfort to the patient is greatly reduced.

Furthermore, with the air-tight component 2 tightly fitted in the side recess 151 of the pivot portion 10, no exterior air would flow into the internal channel 110 of the manifold 1 through interstices between the pivot portion 10 and the switching base 30, thereby preventing the patient from being infected.

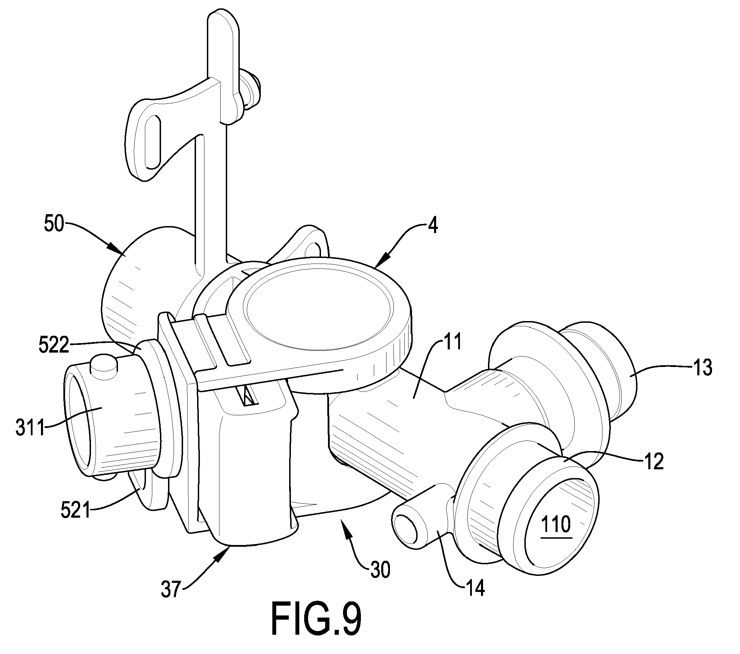

With further reference to Fig. 9, when the main cap 50 caps the distal end of the second connecting tube 312, the sealing film 53 separates the second connecting tube 312 and the internal channel 110 of the manifold 1 from an exterior environment. Thus, with the oxygen tube 13 connecting with the oxygen supply system, the switchable adapter of the present invention is used for supplying oxygen to the patient.

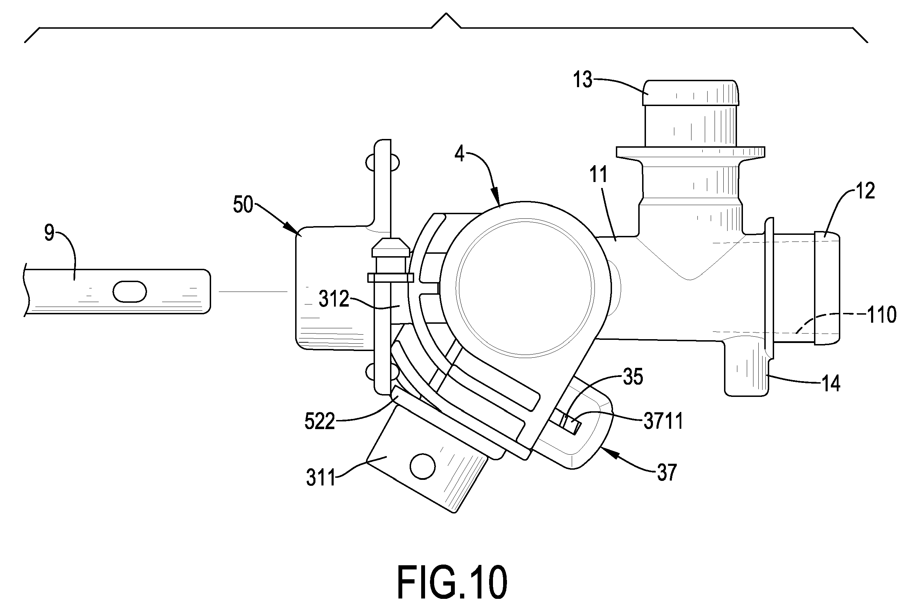

With further reference to Fig. 10, an open suction tube 9 can be further mounted into the through hole 51 of the main cap 50 to push and open the sealing film 53, and protrude into the second connecting tube 312 to suck the sputa out of the trachea of the patient.

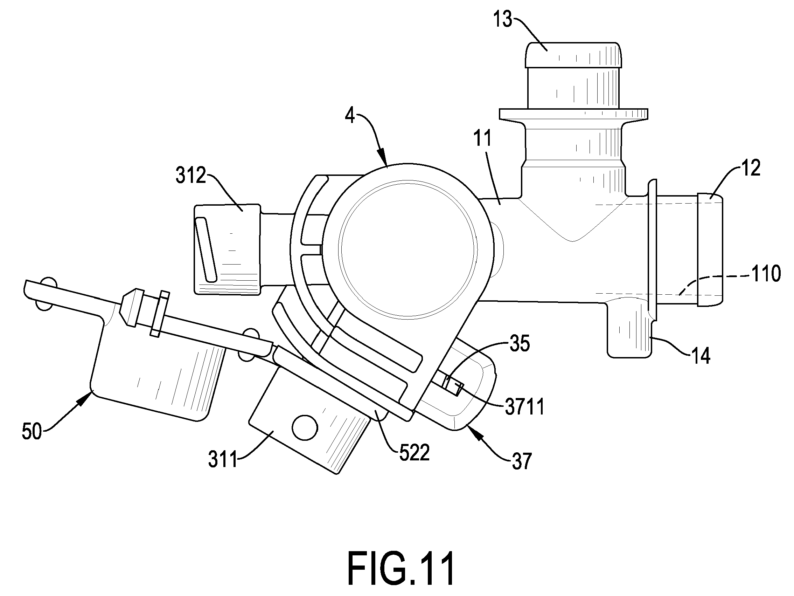

With further reference to Fig. 11, when the main cap 50 is removed from the second connecting tube 312, an endoscope can be directly inserted into the second connecting tube 312 and the trachea of the patient to examine the trachea of the patient.

## Claims

1. A switchable adapter for a suction tube, the switchable adapter comprising a manifold (1), wherein:
the manifold (1) has:
a main tube (11) having an internal channel (110) defined and extending between a front end of the main tube (11) and a rear end of the main tube (11);
at least one branch tube (12, 13, 14) formed on and protruding from the main tube (11), and communicating with the internal channel (110) of the main tube (11); and
a pivot portion (10) attached to the rear end of the main tube (11), and having:
an outer surface (15) attached to the rear end of the main tube (11);
an upper end (16);
a lower end (17);
a side recess (151) formed in the outer surface (15) of the pivot portion (10);
an inner sidewall defined in the side recess (151); and
a mounting hole (1511) formed through the inner sidewall of the pivot portion (10) and communicating with the internal channel (110) of the main tube (11);
the switchable adapter further comprises:
an air-tight component (2) made of resilient material and having a main portion (20) tightly fitted in the side recess (151) of the pivot portion (10), and having a through hole (201) aligning with and communicating with the internal channel (110) of the main tube (11);
a switching base (30) pivotally connected to the pivot portion (10) of the manifold (1) and having
a bottom panel (32);
an arced sidewall (31) formed on and protruding up from the bottom panel (32), and having an inner arc surface andan outer arc surface;
a pivot chamber (33) defined above the bottom panel (32), surrounded by the arced sidewall (31), and receiving the air-tight component (2) and the pivot portion (10) with the lower end (17) of the pivot portion (10) facing the bottom panel (32); and
two connecting tubes (311, 312) including a first connecting tube (311) and a second connecting tube (312), the connecting tubes (311, 312) perpendicularly protruding from the outer arc surface of the arced sidewall (31) and alternatively aligning with the through hole (201) of the air-tight component (2) to communicate with the internal channel (110) of the main tube (11), the first connecting tube (311) and the second connecting tube (312) defining an angle from 30 to 90 degrees; and
a cover (4) mounted on the upper end (16) of the pivot portion (10) of the manifold (1);,
wherein the switching base (30) is configured to pivot relative to the pivot portion (10) of the manifold (1) and about an axis perpendicular to the bottom panel (32) of the switching base (30).

2. The switchable adapter as claimed in claim 1, wherein
the pivot portion (10) of the manifold (1) further has
an upper recess (161) formed in the upper end (16) of the pivot portion (10);
an upper peripheral sidewall defined around the upper recess (161), and having a first notch (162) and a second notch (163);
a lower recess (171) formed in the lower end (17) of the pivot portion (10); and
a lower peripheral sidewall defined around the lower recess (171) and having a first notch (172) and a second notch (173);
the arced sidewall (31) of the switching base (30) further has a mounting slot (314) formed through the arced sidewall (31), disposed beside the bottom panel (32), and selectively corresponding in position to the first or the second notch (172, 173) of the lower peripheral sidewall of the pivot portion (10);
the switching base (30) further has a mounting tube (313) perpendicularly protruding from the first connecting tube (311) and having a distal end; and
the switchable adapter further comprises a pressing assembly (34) mounted on the mounting tube (313) of the switching base (30) and having
a pressing element (37) disposed beside the distal end of the mounting tube (313) of the switching base (30) and having
a force exertion portion (371); and
two guiding sidewalls (372, 373) separately protruding from the force exertion portion (371), and the two guiding sidewalls (372, 373) including
a first guiding sidewall (372) having
an elongated edge corresponding in position to the arced sidewall (31) of the switching base (30); and
a guiding wing (3721) protruding from the elongated edge of the first guiding sidewall (372) and having a rear end disposed apart from the force exertion portion (371) to form a gap (3722) defined between the rear end of the guiding wing (3721) of the first guiding sidewall (372) and the force exertion portion (371); and
a second guiding sidewall (373) having
an elongated edge corresponding in position to the arced sidewall (31) of the switching base (30);
a guiding wing (3731) protruding from the elongated edge of the second guiding sidewall (373) and having a rear end disposed apart from the force exertion portion (371) to form a gap (3732) defined between the rear end of the guiding wing (3731) of the second guiding sidewall (373) and the force exertion portion (371); and
a sliding slot (3733) being elongated and formed through the second guiding sidewall (373); and
a resilient element (35) mounted in the mounting tube (313) of the switching base (30) and having two opposite ends respectively abutting the first connecting tube (311) and the force exertion portion (371) of the pressing element (37) to push the pressing element (37) to slide away from the switching base (30); and
the cover (4) has an inner surface and a limiting protrusion (40) protruding from the inner surface of the cover (4) and protruding in the sliding slot (3733) of the pressing element (37);
wherein the guiding wing (3721) of the first guiding sidewall (372) is mounted through the mounting slot (314) of the arced sidewall (31), protrudes into the lower recess (171) of the pivot portion (10), and selectively engages in the first or the second notch (172, 173) of the lower peripheral sidewall of the pivot portion (10); and
the guiding wing (3731) of the second guiding sidewall (373) protrudes into the upper recess (161) of the pivot portion (10) and selectively engages in the first or the second notch (162, 163) of the upper peripheral sidewall of the pivot portion (10).

3. The switchable adapter as claimed in claim 2, wherein
the bottom panel (32) of the switching base (30) has a guiding slot (321) corresponding in position to and extending parallel to the mounting tube (313); and
the first guiding sidewall (372) of the pressing element (37) further has a sliding protrusion (3723) formed on an inner surface of the first guiding sidewall (372) and protruding in the guiding slot (321) of the bottom panel (32).

4. The switchable adapter as claimed in claim 3, wherein
the pressing element (37) further has a guiding shaft (3711) protruding from the force exertion portion (371) of the pressing element (37) and mounted in the mounting tube (313) of the switching base (30); and
the resilient element (35) is mounted around the guiding shaft (3711) of the pressing element (37).

5. The switchable adapter as claimed in claim 2, wherein
the mounting tube (313) of the switching base (30) communicates with the first connecting tube (311) of the switching base (30);
the pressing assembly (34) further has a pad (36) mounted in the mounting tube (313) of the switching base (30);
the two opposite ends of the resilient element (35) respectively abut the pad (36) and the force exertion portion (371) of the pressing element (37); and
the guiding shaft (3711) of the pressing element (37) is selectively mounted through the pad (36) to protrude into the first connecting tube (311) of the switching base (30).

6. The switchable adapter as claimed in claim 2, 3, 4 or 5 further comprising a cap set (5) selectively capping a distal end of the second connecting tube (312) of the switching base (30), and the cap set (5) including
a main cap (50) capping the distal end of the second connecting tube (312) and having a through hole (51) aligning with the second connecting tube (312); and
a sealing film (53) mounted in the through hole (51) of the main cap (50) and having a peripheral edge partially attached to the main cap (50), such that the sealing film (53) selectively seals the through hole (51) of the main cap (50).

7. The switchable adapter as claimed in claim 6, wherein the cap set (5) further has a collar (522) mounted around the first connecting tube (311) of the switching base (30) and connected with the main cap (50) via a connecting strip (521).

8. The switchable adapter as claimed in claim 7, wherein the air-tight component (2) further has a side tube (21) protruding from the main portion (20), disposed around the through hole (201) of the main portion (20), protruding in the mounting hole (1511) of the pivot portion (10), and communicating with the internal channel (110) of the main tube (11).

## Patentansprüche

1. Schaltbarer Adapter für ein Saugrohr, wobei der schaltbare Adapter einen Verteiler (1) umfasst, wobei:
der Verteiler (1) Folgendes aufweist:
ein Hauptrohr (11), das einen Innenkanal (110) aufweist, der zwischen einem vorderen Ende des Hauptrohrs (11) und einem hinteren Ende des Hauptrohrs (11) definiert ist und verläuft;
wenigstens ein Zweigrohr (12, 13, 14), das an dem Hauptrohr (11) gebildet ist und davon vorsteht und mit dem Innenkanal (110) des Hauptrohrs (11) in Verbindung steht; und
einen Drehzapfenabschnitt (10), der an dem hinteren Ende des Hauptrohrs (11) befestigt ist und Folgendes aufweist:
eine Außenoberfläche (15), die an dem hinteren Ende des Hauptrohrs (11) befestigt ist;
ein oberes Ende (16);
ein unteres Ende (17);
eine seitliche Aussparung (151), die in der Außenoberfläche (15) des Drehzapfenabschnitts (10) gebildet ist;
eine Innenseitenwand, die in der seitlichen Aussparung (151) definiert ist; und
eine Montagebohrung (1511), die durch die Innenseitenwand des Drehzapfenabschnitts (10) gebildet ist und mit dem Innenkanal (110) des Hauptrohrs (11) in Verbindung steht;
wobei der schaltbare Adapter ferner Folgendes umfasst:
eine luftdichte Komponente (2), die aus einem elastischen Material hergestellt ist und einen Hauptabschnitt (20) aufweist, der in die seitliche Aussparung (151) des Drehzapfenabschnitts (10) eingepasst ist, und eine Durchgangsbohrung (201) aufweist, die auf den Innenkanal (110) des Hauptrohrs (11) ausgerichtet ist und mit ihm in Verbindung steht;
eine Schaltgrundplatte (30), die mit dem Drehzapfenabschnitt (10) des Verteilers (1) schwenkbar verbunden ist und Folgendes aufweist:
eine Bodenplatte (32);
eine gebogene Seitenwand (31), die an der Bodenplatte (32) gebildet ist und nach oben von ihr vorsteht und eine Innenbogenfläche und eine Außenbogenfläche aufweist;
eine Drehzapfenkammer (33), die über der Bodenplatte (32) definiert ist, die von der gebogenen Seitenwand (31) umgeben ist und die luftdichte Komponente (2) und den Drehzapfenabschnitt (10) aufnimmt, wobei das untere Ende (17) des Drehzapfenabschnitts (10) der Bodenplatte (32) zugewandt ist; und
zwei Verbindungsrohre (311, 312), die ein erstes Verbindungsrohr (311) und ein zweites Verbindungsrohr (312) enthalten, wobei die Verbindungsrohre (311, 312) von der Außenbogenfläche der gebogenen Seitenwand (31) senkrecht vorstehen und alternativ auf die Durchgangsbohrung (201) der luftdichten Komponente (2) ausgerichtet sind, um mit dem Innenkanal (110) des Hauptrohrs (11) in Verbindung zu stehen, wobei das erste Verbindungsrohr (311) und das zweite Verbindungsrohr (312) einen Winkel von 30 bis 90 Grad definieren; und
eine Abdeckung (4) die an dem oberen Ende (16) des Drehzapfenabschnitts (10) des Verteilers (1) montiert ist;
wobei die Schaltgrundplatte (30) dafür konfiguriert ist, relativ zu dem Drehzapfenabschnitt (10) des Verteilers (1) und um eine Achse senkrecht zu der Bodenplatte (32) der Schaltgrundplatte (30) zu schwenken.

2. Schaltbarer Adapter gemäß Anspruch 1, wobei
der Drehzapfenabschnitt (10) des Verteilers (1) ferner Folgendes aufweist:
eine obere Aussparung (161), die in dem oberen Ende (16) des Drehzapfenabschnitts (10) gebildet ist;
eine obere Umfangsseitenwand, die um die obere Aussparung (161) definiert ist und eine erste Kerbe (162) und eine zweite Kerbe (163) aufweist;
eine untere Aussparung (171), die in dem unteren Ende (17) des Drehzapfenabschnitts (10) gebildet ist; und
eine untere Umfangsseitenwand, die um die untere Aussparung (171) definiert ist und eine erste Kerbe (172) und eine zweite Kerbe (173) aufweist,
wobei die gebogene Seitenwand (31) der Schaltgrundplatte (30) ferner einen durch die gebogene Seitenwand (31) gebildeten Montageschlitz (314) aufweist, der neben der Bodenplatte (32) angeordnet ist und dessen Position wahlweise der der ersten oder der zweiten Kerbe (172, 173) der unteren Umfangsseitenwand des Drehzapfenabschnitts (10) entspricht;
wobei die Schaltgrundplatte (30) ferner ein Montagerohr (313) aufweist, das von dem ersten Verbindungsrohr (311) senkrecht vorsteht und ein distales Ende aufweist; und
wobei der schaltbare Adapter fernen eine Druckanordnung (34) umfasst, die an dem Montagerohr (313) der Schaltgrundplatte (30) angebracht ist und Folgendes aufweist:
ein Druckelement (37), das neben dem distalen Ende des Montagerohrs (313) der Schaltgrundplatte (30) angeordnet ist und Folgendes aufweist:
einen Kraftbeaufschlagungsabschnitt (371); und
zwei Führungsseitenwände (372, 373), die von dem Kraftbeaufschlagungsabschnitt (371) getrennt vorstehen, und wobei die zwei Führungsseitenwände (372, 373) Folgendes enthalten:
eine erste Führungsseitenwand (372), die Folgendes aufweist:
einen langgestreckten Rand, dessen Position der der gebogenen Seitenwand (31) der Schaltgrundplatte (30) entspricht; und
einen Führungsflügel (3721), der von dem langgestreckten Rand der ersten Führungsseitenwand (372) vorsteht und ein hinteres Ende aufweist, das von dem Kraftbeaufschlagungsabschnitt (371) beabstandet angeordnet ist, um einen Zwischenraum (3722) zu bilden, der zwischen dem hinteren Ende des Führungsflügels (3721) der ersten Führungsseitenwand (372) und dem Kraftbeaufschlagungsabschnitt (371) definiert ist; und
eine zweite Führungsseitenwand (373), die Folgendes aufweist:
einen langgestreckten Rand, dessen Position der der gebogenen Seitenwand (31) der Schaltgrundplatte (30) entspricht;
einen Führungsflügel (3731), der von dem langgestreckten Rand der zweiten Führungsseitenwand (373) vorsteht und ein hinteres Ende aufweist, das von dem Kraftbeaufschlagungsabschnitt (371) beabstandet angeordnet ist, um einen Zwischenraum (3732) zu bilden, der zwischen dem hinteren Ende des Führungsflügels (3731) der zweiten Führungsseitenwand (373) und dem Kraftbeaufschlagungsabschnitt (371) definiert ist; und
einen Gleitschlitz (3733), der langgestreckt ist und durch die zweite Führungsseitenwand (373) gebildet ist; und
ein elastisches Element (35), das in dem Montagerohr (313) der Schaltgrundplatte (30) montiert ist und zwei gegenüberliegende Enden aufweist, die an dem ersten Verbindungsrohr (311) bzw. an dem Kraftbeaufschlagungsabschnitt (371) des Druckelements (37) anliegen, um das Druckelement (37) zu schieben, damit es von der Schaltgrundplatte (30) weggleitet; und
wobei die Abdeckung (4) eine Innenoberfläche und einen Begrenzungsvorsprung (40), der von der Innenoberfläche der Abdeckung (4) vorsteht und in den Gleitschlitz (3733) des Druckelements (37) vorsteht, aufweist;
wobei der Führungsflügel (3721) der ersten Führungsseitenwand (372) durch den Montageschlitz (314) der gebogenen Seitenwand (31) montiert ist, in die untere Aussparung (171) des Drehzapfenabschnitts (10) vorsteht und in der ersten oder in der zweiten Kerbe (172, 173) der unteren Umfangsseitenwand des Drehzapfenabschnitts (10) wahlweise in Eingriff ist; und
wobei der Führungsflügel (3731) der zweiten Führungsseitenwand (373) in die obere Aussparung (161) des Drehzapfenabschnitts (10) vorsteht und in der ersten oder in der zweiten Kerbe (162, 163) der oberen Umfangsseitenwand des Drehzapfenabschnitts (10) wahlweise in Eingriff ist.

3. Schaltbarer Adapter gemäß Anspruch 2, wobei
die Bodenplatte (32) der Schaltgrundplatte (30) einen Führungsschlitz (321) aufweist, dessen Position der des Montagerohrs (313) entspricht und der parallel zu diesem verläuft; und
die erste Führungsseitenwand (372) des Druckelements (37) ferner einen Gleitvorsprung (3723) aufweist, der an einer Innenoberfläche der ersten Führungsseitenwand (372) gebildet ist und in den Führungsschlitz (321) der Bodenplatte (32) vorsteht.

4. Schaltbarer Adapter gemäß Anspruch 3, wobei
das Druckelement (37) ferner einen Führungsschaft (3711) aufweist, der von dem Kraftbeaufschlagungsabschnitt (371) des Druckelements (37) vorsteht und in dem Montagerohr (313) der Schaltgrundplatte (30) montiert ist; und
das elastische Element (35) um den Führungsschaft (3711) des Druckelements (37) montiert ist.

5. Schaltbarer Adapter gemäß Anspruch 2, wobei
das Montagerohr (313) der Schaltgrundplatte (30) mit dem ersten Verbindungsrohr (311) der Schaltgrundplatte (30) in Verbindung steht;
die Druckanordnung (34) ferner ein Druckstück (36) aufweist, das in dem Montagerohr (313) der Schaltgrundplatte (30) montiert ist;
die zwei gegenüberliegenden Enden des elastischen Elements (35) an dem Druckstück (36) bzw. an dem Kraftbeaufschlagungsabschnitt (371) des Druckelements (37) anliegen; und
der Führungsschaft (3711) des Druckelements (37) wahlweise durch das Druckstück (36) montiert ist, um in das erste Verbindungsrohr (311) der Schaltgrundplatte (30) vorzustehen.

6. Schaltbarer Adapter gemäß Anspruch 2, 3, 4 oder 5, der ferner einen Kappensatz (5) umfasst, der wahlweise ein distales Ende des zweiten Verbindungsrohrs (312) der Schaltgrundplatte (30) abdeckt, wobei der Kappensatz (5) Folgendes enthält:
eine Hauptkappe (50), die das distale Ende des zweiten Verbindungsrohrs (312) abdeckt und eine Durchgangsbohrung (51) aufweist, die auf das zweite Verbindungsrohr (312) ausgerichtet ist; und
einen Abdichtfilm (53), der in der Durchgangsbohrung (51) der Hauptkappe (50) montiert ist und einen Umfangsrand aufweist, der in der Weise teilweise an der Hauptkappe (50) befestigt ist, dass der Abdichtfilm (53) die Durchgangsbohrung (51) der Hauptkappe (50) wahlweise abdichtet.

7. Schaltbarer Adapter gemäß Anspruch 6, wobei der Kappensatz (5) ferner einen Ring (522) aufweist, der um das erste Verbindungsrohr (311) der Schaltgrundplatte (30) montiert ist und über einen Verbindungsstreifen (521) mit der Hauptkappe (50) verbunden ist.

8. Schaltbarer Adapter gemäß Anspruch 7, wobei die luftdichte Komponente (2) ferner ein von dem Hauptabschnitt (20) vorstehendes Seitenrohr (21) aufweist, das um die Durchgangsbohrung (201) des Hauptabschnitts (20) angeordnet ist, in die Montagebohrung (1511) des Drehzapfenabschnitts (10) vorsteht und mit dem Innenkanal (110) des Hauptrohrs (11) in Verbindung steht.

## Revendications

1. Adaptateur commutable pour un tube d'aspiration, l'adaptateur commutable comprenant un collecteur (1), dans lequel :
le collecteur (1) comporte :
un tube principal (11) ayant un canal interne (110) défini et s'étendant entre une extrémité avant du tube principal (11) et une extrémité arrière du tube principal (11) ;
au moins un tube de branchement (12, 13, 14) formé sur et faisant saillie du tube principal (11) et communiquant avec le canal interne (110) du tube principal (11) ; et
une portion de pivot (10) fixée à l'extrémité arrière du tube principal (11) et comportant :
une surface extérieure (15) fixée à l'extrémité arrière du tube principal (11) ;
une extrémité supérieure (16) ;
une extrémité inférieure (17) ;
un évidement latéral (151) formé dans la surface extérieure (15) de la portion de pivot (10) ;
une paroi intérieure définie dans l'évitement latéral (151) ; et
un trou de montage (1511) formé à travers la paroi latérale intérieure de la portion de pivot (10) et communiquant avec le canal interne (110) du tube principal (11);
l'adaptateur commutable comprend en outre :
un composant hermétique (2) constitué de matériau élastique et ayant une portion principale (20) étroitement ajustée dans l'évidement latéral (151) de la portion de pivot (10),
et comportant un trou passant (201) s'alignant et communiquant avec le canal interne (110) du tube principal (11) ;
une base de commutation (30) connectée de manière pivotante à la portion de pivot (10) du collecteur (1) et comportant
un panneau de fond (32) ;
une paroi latérale en arc (31) formée sur et faisant saillie vers le haut à partir du panneau de fond (32) et ayant une surface d'arc intérieure et une surface d'arc extérieure ;
une chambre de pivot (33) définie au-dessus du panneau de fond (32), entourée par la paroi latérale en arc (31), et recevant le composant hermétique (2) et la portion de pivot (10) avec l'extrémité inférieure (17) de la portion de pivot (10) faisant face au panneau de fond (32) ; et
deux tubes de connexion (311, 312) comprenant un premier tube de connexion (311) et un deuxième tube de connexion (312), les tubes de connexion (311, 312) faisant saillie perpendiculairement à partir de la surface d'arc extérieure de la paroi latérale en arc (31), et s'alignant alternativement avec le trou passant (201) du composant hermétique (2) pour communiquer avec le canal interne (110) du tube principal (11), le premier tube de connexion (311) et le deuxième tube de connexion (312) définissant un angle de 30 à 90 degrés ; et
un couvercle (4) monté sur l'extrémité supérieure (16) de la portion de pivot (10) du collecteur (1) ;
dans lequel la base de commutation (30) est configurée pour pivoter par rapport à la portion de pivot (10) du collecteur (1) et autour d'un axe perpendiculaire au panneau de fond (32) de la base de commutation (30).

2. Adaptateur commutable selon la revendication 1, dans lequel
la portion de pivot (10) du collecteur (1) comporte en outre
un évidement supérieur (161) formé dans l'extrémité supérieure (16) de la portion de pivot (10) ;
une paroi latérale périphérique supérieure définie autour de l'évidement supérieur (161), et comportant une première encoche (162) et une deuxième encoche (163) ;
un évidement inférieur (171) formé dans l'extrémité inférieure (17) de la portion de pivot (10) ; et
une paroi latérale périphérique inférieure définie autour de l'évidement inférieur (171), et comportant une première encoche (172) et une deuxième encoche (173) ;
la paroi latérale en arc (31) de la base de commutation (30) comporte en outre une fente de montage (314) formée à travers la paroi latérale en arc (32), disposée à côté du panneau de fond (32) et correspondant de manière sélective en position à la première ou la deuxième encoche (172, 173) de la paroi latérale périphérique inférieure de la portion de pivot (10) ;
la base de commutation (30) a en outre un tube de montage (313) faisant saillie perpendiculairement à partir du premier tube de connexion (311) et ayant une extrémité distale ; et
l'adaptateur commutable comprend en outre un ensemble de pressage (34) monté sur le tube de montage (313) de la base de commutation (30) et comportant un élément de pressage (37) disposé à côté de l'extrémité distale du tube de montage (313) de la base de commutation (30) et comportant
une portion d'exercice de force (371) ; et
deux parois latérales de guidage (372, 373) faisant saillie séparément à partir de la portion d'exercice de force (371), et les deux parois latérales de guidage (372, 373) comprenant une première paroi latérale de guidage (372) comportant
un bord allongé correspondant en position à la paroi latérale en arc (31) de la base de commutation (30) ; et
une aile de guidage (3721) faisant saillie à partir du bord allongé de la première paroi latérale de guidage (372) et ayant une extrémité arrière disposée à distance de la portion d'exercice de force (371) pour former un espace (3722) défini entre l'extrémité arrière de l'aile de guidage (3721) de la première paroi latérale de guidage (372) et la portion d'exercice de force (371) ; et
une deuxième paroi latérale de guidage (373) comportant
un bord allongé correspondant en position à la paroi latérale en arc (31) de la base de commutation (30) ;
une aile de guidage (3731) faisant saillie à partir du bord allongé de la deuxième paroi latérale de guidage (373) et ayant une extrémité arrière disposée à distance de la portion d'exercice de force (371) pour former un espace (3732) défini entre l'extrémité arrière de l'aile de guidage (3731) de la deuxième paroi latérale de guidage (373) et la portion d'exercice de force (371) ; et
une fente de coulissement (3733) étant allongée et formée à travers la deuxième paroi latérale de guidage (373) ; et
un élément élastique (35) monté dans le tube de montage (313) de la base de commutation (30) et ayant deux extrémités opposées jouxtant respectivement le premier tube de connexion (311) et la portion d'exercice de force (371) de l'élément de pressage (37) pour pousser l'élément de pressage (37) pour coulisser en s'éloignant de la base de commutation (30) ; et
le couvercle (4) a une surface intérieure et une saillie de limitation (40) faisant saillie à partir de la surface intérieure du couvercle (4) et faisant saillie dans la fente de coulissement (3733) de l'élément de pressage (37) ;
dans lequel l'aile de guidage (3721) de la première paroi latérale de guidage (372) est montée à travers la fente de montage (314) de la paroi latérale en arc (31), fait saillie dans l'évidement inférieur (171) de la portion de pivot (10) et s'engage de manière sélective dans la première ou la deuxième encoche (172, 173) de la paroi latérale périphérique inférieure de la portion de pivot (10) ; et
l'aile de guidage (3731) de la deuxième paroi latérale de guidage (373) fait saillie dans l'évidement supérieur (161) de la portion de pivot (10) et s'engage de manière sélective dans la première ou la deuxième encoche (162, 163) de la paroi latérale périphérique supérieure de la portion de pivot (10).

3. Adaptateur commutable selon la revendication 2, dans lequel
le panneau de fond (32) de la base de commutation (30) comporte une fente de guidage (321) correspondant en position à et s'étendant parallèlement au tube de montage (313) ; et
la première paroi latérale de guidage (372) de l'élément de pressage (37) comporte en outre une saillie de coulissement (3723) formée sur une surface intérieure de la première paroi latérale de guidage (372) et faisant saillie dans la fente de guidage (321) de la paroi de fond (32).

4. Adaptateur commutable selon la revendication 3, dans lequel
l'élément de pressage (37) a en outre un arbre de guidage (3711) faisant saillie à partir de la portion d'exercice de force (371) de l'élément de pressage (37) et monté dans le tube de montage (313) de la base de commutation (30) ; et
l'élément élastique (35) est monté autour de l'arbre de guidage (3711) de l'élément de pressage (37).

5. Adaptateur commutable selon la revendication 2, dans lequel
le tube de montage (313) de la base de commutation (30) communique avec le premier tube de connexion (311) de la base de commutation (30) ;
l'ensemble de pressage (34) a en outre un bloc (36) monté dans le tube de montage (313) de la base de commutation (30) ;
les deux extrémités opposées de l'élément de pressage (35) jouxtent respectivement le bloc (36) et la portion d'exercice de force (371) de l'élément de pressage (37) ; et
l'arbre de guidage (3711) de l'élément de pressage (37) est monté de manière sélective à travers le bloc (36) pour faire saillie dans le premier tube de connexion (311) de la base de commutation (30).

6. Adaptateur commutable selon la revendication 2, 3, 4 ou 5, comprenant en outre un ensemble de capuchon (5) recouvrant de manière sélective une extrémité distale du deuxième tube de connexion (312) de la base de commutation (30), et l'ensemble de capuchon (5) comprenant
un capuchon principal (50) recouvrant l'extrémité distale du deuxième tube de connexion (312) et ayant un trou passant (51) s'alignant avec le deuxième tube de connexion (312) ; et
un film de scellement (53) monté dans le trou passant (51) du capuchon principal (50) et ayant un bord périphérique partiellement attaché au capuchon principal (50), de manière que le film de scellement (53) scelle de manière sélective le trou passant (51) du capuchon principal (50).

7. Adaptateur commutable selon la revendication 6, dans lequel l'ensemble de capuchon (5) comporte en outre un collier (522) monté autour du premier tube de connexion (311) de la base de commutation (30) et connecté avec le capuchon principal (50) par l'intermédiaire d'une bande de connexion (521).

8. Adaptateur commutable selon la revendication 7, dans lequel le composant hermétique (2) comprend en outre un tube latéral (21) faisant saillie à partir de la portion principale (20), disposé autour du trou passant (201) de la portion principale (20), faisant saillie dans le trou de montage (1511) de la portion de pivot (10) et communiquant avec le canal interne (110) du tube principal (11).
